Europäisches Patentamt

European Patent Office

Office européen des brevets

(11) Publication number:

**0 351 921**
**A2**

# EUROPEAN PATENT APPLICATION

(21) Application number: **89201885.4**

(22) Date of filing: **19.07.89**

(51) Int. Cl.⁴: **A01K 67/00 , C12N 15/85**

(30) Priority: **22.07.88 US 223182**

(43) Date of publication of application:
**24.01.90 Bulletin 90/04**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Dixon, Richard A. F.**
**758 Hartley Drive**
**Lansdale, PA 19446(US)**
Inventor: **Patchett, Arthur A.**
**1090 Minisink Way**
**Westfield New Jersey 07090(US)**
Inventor: **Sigal, Irving S.**

**deceased(US)**
Inventor: **Strader, Catherine D.**
**119 Morningside Road**
**Verona New Jersey 07044(US)**

(74) Representative: **Hesketh, Alan, Dr. et al**
**European Patent Department Merck & Co.,**
**Inc. Terlings Park Eastwick Road**
**Harlow Essex, CM20 2QR(GB)**

(54) **Modified beta adrenergic receptor.**

(57) Modified Beta Adrenergic Receptors having the amino acid residue in position 113 substituted by certain other amino acids have been synthesized. These modified receptors interact with novel ligands that do not react with the natural Asp 113 containing receptors. Transgenic animals containing this modified receptor will respond to novel ligands which will not interact with the normal receptor.

RELATED APPLICATION

The present application is a continuation-in-part of our copending U.S. Patent Application Serial No. 223,182, filed 22 July, 1988.

## MODIFIED BETA ADRENERGIC RECEPTOR

### BACKGROUND OF THE INVENTION

Although hormone and drug receptors have been studied for most of this century it was the recent development of ligand-binding techniques that has permitted direct assay of the binding function of receptor macromolecules. Of the membrane bound hormone and drug receptors considerable attention has been directed to the adenylyl cyclase-coupled beta-adrenergic receptors because of their ubiquity and the diversity of the physiological responses they mediate; the close coupling of the receptors to a well-defined biochemical effector unit, the enzyme adenylyl cyclase, which has facilitated the use of the beta-adrenergic receptor ($\beta$AR) adenylyl cyclase system as a model for studying the biochemical mechanisms of hormone receptor-effector coupling; and the therapeutic consequences of stimulation or blockade of these receptors by pharmacological means, which have implications for the therapy of a wide variety of human illnesses and to control the growth and metabolism of animals.

The $\beta$-adrenergic receptor ($\beta$AR) is a member of a large class of hormone receptors that are located at the plasma membrane and that exert their intracellular effects through an interaction with guanine nucleotide binding proteins (G proteins). Different G-protein-linked receptors recognize different ligands as agonists and stimulate distinct classes of G proteins. In the case of $\beta$AR, upon binding catecholamine agonists, the receptor catalyzes the formation of the GTP complex of $G_s$, which in turn stimulates adenylyl cyclase activity. $\beta$AR antagonists appear to act by competing with agonists for binding to the receptor and failing to stimulate adenylyl cyclase. Two subtypes of $\beta$AR occurs in mammalian tissue. The $\beta_2$ subtype, found to predominate in lung tissue, binds epinephrine preferentially to norepinephrine, whereas the $\beta_1$ subtype, which predominates in heart tissue, binds both agonists with similar affinities.

Dixon et al., Nature 321, 75-79, 1 May 1986, reported the cloning of the mammalian B$_2$AR gene, its cDNA sequence and its predicted amino acid sequence. Deletion mutagenesis experiments have demonstrated that the binding site of the $\beta$-adrenergic receptor involves the hydrophobic core of the protein [Dixon et al. (1987) Nature 326, 73-77]. Single amino acid replacements for the conserved Asp 79 and Asp 113 within this putative transmembrane region had profound effects on the ability of the receptor to bind radiolabeled ligands [Strader et al. (1987) Proc. Natl. Acad. Sci. 84, 4384-4388].

The use of $\beta$AR agonists to enhance food efficiency and carcass composition of food animals is a known technique, $\beta$-agonists and their Effects On Animal Growth and Carcass Quality, J.P. Hanrahan, ed., Elsevier Appl. Sci. Press, New York, 1987. The presence of even residual quantities of these agonists in meat, however, is undesirable because of possible physiological effects on the natural $\beta$AR of human consumers.

### SUMMARY OF THE INVENTION

It has now been found that substitution of the aspartate-113 residue of the $\beta$AR receptor with arginine (Arg), asparagine (Asn), cysteine (Cys), glutamic acid (Glu), histidine (His), or lysine (Lys) yields a modified $\beta$AR that responds poorly or not at all to classical adrenergic ligands such as catecholamines but does respond to ligands that do not react with unmodified or natural $\beta$AR. The modified $\beta$AR can be expressed in transgenic animals using muscle specific promoters so that the animals grow in response to novel ligands. The $\beta$ARs and coupled metabolic systems of unmodified species including man, as well as the unmodified $\beta$ARs of the transgenic animal, are unaffected by the ingestion of these ligands.

### OBJECTS OF THE INVENTION

It is, accordingly, an object of the present invention to provide a novel $\beta$AR that reacts with ligands that do not react with the natural (wild-type) $\beta$AR. Another object is to provide vectors containing the genes that expresses these novel $\beta$ARs. Still another method is to provide transgenic animals containing these novel $\beta$ARs. Yet another object is to provide a method for enhancing food efficiency and carcass composition of food animals by means of $\beta$AR agonists that do not react with the $\beta$AR of human consumers. These and other objects of the present invention will be apparent from the following description.

## DETAILED DESCRIPTION OF THE INVENTION

The present invention is based on the finding that a change in a specific amino acid of the $\beta$AR produces a modified receptor that will interact with ligands that do not react with the unmodified or natural $\beta$AR. The specific amino acid of the $\beta$AR that is changed according to the present invention is the aspartic acid residue in position 113.

This aspartic acid residue can be substituted with another amino acid to provide a novel modified or mutant receptor that responds to ligands that do not react with unmodified or natural (wild-type) $\beta$AR. By way of specific exemplification, amino acids that may be substituted for the aspartic avoid residue at position 113 are the following: arginine, asparagine, cysteine, glutamic acid, histidine and lysine.

According to the invention described herein we have engineered the $\beta$AR by replacing the Asp[113] residue with one of the foregoing amino acids, and we have identified compounds which act as agonists for some of these mutant receptors while stimulating the wild type receptor poorly or not at all. Thus, alterations in the design of the ligand binding pocket of the receptor, accompanied by complementary alterations in the functional groups on the ligand have resulted in the creation of new positive interactions between a receptor and a ligand

The following examples illustrate the present inventions without, however, limiting the same thereto.

## EXAMPLE 1

### Mutagenesis of the $\beta$AR gene.

The cloning of the cDNA for the hamster $\beta$-adrenergic receptor (BAR) has been described by Dixon et al., Nature (1986) supra. For the introduction of mutations into the $\beta$AR, the cDNA was cloned into the EcoRI site of M13 mp8 and single stranded phage DNA containing the noncoding strand of the $\beta$AR cDNA was isolated by standard procedures as described by Maniatis et al., (1982) Molecular Cloning: A Laboratory Manual (Cold Spring Harbor Laboratory, Cold Spring Harbor, N.Y.). Other standard cloning techniques were performed as described by the former Manual and would be easily reproduced by anyone trained in molecular biology. Oligonucleotides 19 nucleotides in length complementary to the phage DNA surrounding the codon for Asp[113] were synthesized using an Applied Biosystems Model 3A DNA synthesizer. The oligonucleotides contained mismatched positions in order to convert the codon for Asp[113] (GAT) to either Arg (GACTTCCATTCGTGTGTTA), Cys (GACTTCCATTTGTGTGTTA), Glu (GACTTCCATTGAGGTGTTA, His (GACTTCCATTCATGTGTTA), Lys (GACTTCCATTAAGGTGTTA), Gln (GACTTCCATTCAGGTGTTA), Ser (GACTTCCATTTCTGTGTTA), Thr (GACTTCCATTACTGTGTTA), Phe (GACTTCCATTTTTGTGTTA), Ala (GACTTCCATTGCTGTGTTA). The oligonucleotides were annealed to the phage DNA for priming and extended using Klenow DNA polymerase and T4 DNA ligase. The double stranded DNA was digested with AccI and KpnI and the mutated fragment was ligated to the mammalian expression vector pSVHAC [Dixon et al., Nature (1987), supra] which was also digested with AccI and KpnI. pSVBAC contains the full length hamster $\beta_2$AR cDNA cloned into the EcoRI site of pSVL. pSVL contains the SV40 origin of DNA replication and a modified RNA splicing sequence [Okayama et al. (1983) Mol. Cell. Biol. 3, 280-289]. The $\beta$AR gene in this vector utilizes the SV40 early promoter, the modified splice signal, and the $\beta$AR polyadenylation site for expression. The ligated mixture was transformed into E>. coli strain DH5 (Bethesda Research Laboratories) and ampicillin resistant colonies were screened by filter hybridization using the oligonucleotide corresponding to the mutation as the hybridization probe. Plasmids were isolated from several hybridization positive colonies and mapped to insure the proper DNA insert was contained in the clone. The mutations were resolved by retransformation of the plasmid DNA into E. coli. Mutant plasmid containing colonies were again isolated by filter hybridization as above. Plasmid DNA was isolated from overnight cultures of cells by alkaline lysis and twice banding in CsCl-ethidium bromide equilibrium density gradients. The region of each mutant plasmid which was subcloned into the vector was sequenced to confirm the identity of the mutation. The purity of the plasmid preparation was tested by retransformation of the DNA into E. coli followed by colony hybridization. Of the 100 colonies examined for each plasmid, all were positive by hybridization. A neomycin drug resistance marker was added to the pSVHAC plasmid containing each of the position 113 mutations. The Klenow DNA polymerase, blunt-ended EcoRI fragment containing the tk-neo cassette from plasmid pCMVIE-AKI DHFR (Silberklang et al., 1987, in "Modern Approaches to Animal Cell Technology" eds. R. E. Spier and J. B. Griffiths, Butterworth and Co.,

3

U.K., pp. 199-214) was subcloned into the blunt-ended HindIII site of each of the mutant containing plasmids. Plasmid DNA was isolated as above and confirmed by restriction mapping.

## EXAMPLE 2

### Expression in mammalian cells.

For the establishment of clonal cell lines expressing mutant receptors, the plasmid DNAs were transfected into mouse L cells by the $CaPO_4$ procedure. The DNA (15mg) in HEPES buffered saline was precipitated with 125 mM $CaCl_2$ at room temperature for 20 min. The precipitate was added to the media covering subconfluent monolayers of mouse L cells in 100 mm petri dishes and incubated at 37°C for 4 hrs. The media was removed and the cells were washed once with Dulbecco's modified minimal essential medium (DMEM). DMEM containing 15% glycerol added to the monolayer for 1 min followed by three washes with DMEM. DMEM containing 10% fetal bovine serum was then added to the monolayers and the cells were incubated at 37°C for 12-18 hrs. The medium was changed to DMEM containing 10% fetal bovine serum and 500 mg/ml genticin to select for cells containing the expression vector. The cells were then incubated at 37°C for 1-2 weeks to allow genticin-resistant colonies to form. Individual colonies were isolated and expanded into cell lines.

L cells expressing the wild type receptor were identified by $^{125}$I-cyanopindolol ($^{125}$I-CYP) binding as described below. L cells expressing mutant receptors which did not bind ligands were identified by indirect immunofluorescence using a monoclonal antibody which recognizes the $\beta$AR. The antibody was raised against a peptide corresponding to a region of the proposed 3rd intracellular loop of the hamster $\beta$AR, with the sequence Y-A-K-R-Q-L-Q-K-I-D-K-S-E-G-R, Zemcik et al. (1988) Biochem. J. 251, 333-339. Cell monolayers were washed in phosphate buffered saline (PBS), fixed in 3.7% formalin, 1% ethyldimethyldiaminopropyl carbodiimide in PBS for 10 min at 23°C. After permeabilization in 0.1% Triton X-100 for 5 min, cells were treated with 4 mg/ml of goat gamma globulin for 1 hr at 23°C to reduce nonspecific alterations, then washed and incubated with the monoclonal antibody at 0.1 mg/ml for 1 hr at 23°C. After further washing in PBS, cells were incubated with FITC-conjugated goat anti-mouse IgG at a 1:50 dilution for 1 hr at 23°C. Cells were then washed extensively in PBS and the fluorescence observed using a Leitz Diaplan fluorescence microscope.

## EXAMPLE 3

### Membrane Preparation.

COS-7 cell monolayers were washed 3 times in PBS, scraped from the flask with a rubber policeman, and centrifuged at 2000 x g for 5 min. The cell pellet was resuspended in 75 mM Tris, pH 7.4, 12.5 mM $MgCl_2$, 1.5 mM EDTA (TME buffer) at a concentration of 108 cells/ml, then diluted 1:5 with water and lysed by freezing in liquid $N_2$ and thawing with vigorous vortexing. Nuclei were removed by centrifugation over a cushion of 60% sucrose at 2500 x g for 15 min. The upper layer was collected, membranes pelleted by centrifugation at 40,000 x g for 15 min, and resuspended in TME at a protein concentration of 1-2 mg/ml.

Membranes were prepared from L cell monolayers by hypotonic lysis in 10 mM Tris, pH 7.5 for 10 min at 4°C. Cells were then scraped from the flask in the hypotonic buffer, membranes pelleted by centrifugation at 40,000 x g for 15 min, and resuspended at a protein concentration of 0.5-2 mg/ml in TME buffer.

## EXAMPLE 4

### Receptor Assays.

4

125I-CYP binding to membranes was measured in a final volume of 250 ul of TME buffer, containing 10-20 mg of membrane protein and 225 pM 125I-CYP for 90 min at 23°C, as previously described Dixon et al. (1987), supra. Membranes were collected by filtration on GF/C glass fiber filters and bound 125I-CYP was detected with a gamma counter. Nonspecific binding, measured in the presence of 10 mM alprenolol, was subtracted from the total binding.

Adenylyl cyclase activity was determined by the method of Saloman et al., (1974) Anal. Biochem. 58, 541-548. Briefly, 20 ml of TME buffer containing 5-20 mg of membrane protein was mixed with 30 ml of a solution containing 7 mM phosphoenolpyruvate, 0.3 mM ATP (with $10^6$ cpm of a-$^{32}$P-ATP), 0.3 mM cAMP, 0.1 mM GTP, 1.5 units of mykokinase, and 0.3 units of pyruvate kinase. The reaction was allowed to proceed for 30 min at 30°C before it was quenched at 4°C with an excess of unlabeled ATP. The $^{32}$P-cAMP produced was determined by Dowex and alumina chromatography, as described by Salomon et al., supra. For stimulation of the enzyme, test compounds were included in the assay at the concentrations from $10^{-10}$ to $10^{-3}$ M. Maximal stimulation was achieved with 10 mM NaF.

EXAMPLE 5

Data Analysis.

Activation constant ($K_{act}$) values for adenylyl cyclase stimulation were obtained by computer assisted nonlinear regression analysis of curves generated in the presence of increasing concentrations of compounds. The Michaelis-Menton equation was used: $V = V_{max}[A]/K_{act} + [A]$, where V is the level of cyclase stimulation (in pmol $^{32}$P-cAMP/mg protein/min) measured at a given agonist concentration [A], and $V_{max}$ is the calculated maximal stimulation at infinite agonist concentration. In cases where the stimulation curve did not reach a plateau at experimentally assessible concentrations of a compound, Vmax was assumed to be equal to the maximal stimulation achieved with 10 mM NaF, as explained in the legend to Table 1.

EXAMPLE 6

Protein Immunoblotting.

COS-7 membranes containing 1 mg of membrane protein were pelleted by centrifugation at 15,000 x g for 30 min and resuspended at a concentration of 10-20 mg/ml in 65 mM Tris, pH6.3, 3% SDS, 10% glycerol, 5% 2-mercaptoethanol. Proteins were denatured at 4°C for 1 hr and separated on 10% polyacrylamide gels by the method of Laemmli (1970) Nature 117, 680-685. After electrophoresis, proteins were transferred to nitrocellulose and the immunoreactive receptor detected by immunoblotting Towbin et al., (1979) Proc. Natl. Acad. Sci. (USA) 76, 2974-2978. The antibody used in immunoblotting was raised to a peptide corresponding to the C-terminus of the hamster βAR, with the sequence C-L-D-S-Q-G-R-N-Nle-S-T-N-D-S-P-L, used at a serum dilution of 1:1000, followed by 125I-Protein A at a final concentration of $10^6$ cpm/ml.

RESULTS

The expression of mutant βARs having either Arg, Asn, Cys, Glu, His, Gln, Ser, Thr, Phe, Ala, or Lys at position 113 was first assessed in membranes from COS-7 cells by protein immunoblotting. Transfection of these cells with the wild-type (natural) receptor resulted in the production of an immunoreactive protein which migrates with an apparent molecular weight of 68 kDa. The appearance of this fully glycosylated form of the receptor was previously determined to indicate proper folding and processing of the protein in the cell membrane (Strader et al., supra). All of the mutant βARs having substitutions at position 113 comigrated with the wild type βAR, suggesting that these amino acid substitutions did not affect the folding of the protein in the cell membrane. However, 125I-CYP binding could be measured only for the wild-type

5

receptor, as neither [Arg[113]]$\beta$AR, [Asn[113]]$\beta$AR, [Cys[112]]$\beta$AR, [Glu[113]]$\beta$AR, [His[113]]$\beta$AR, [Gln[113]]$\beta$AR, [Ser[113]]-$\beta$AR, [Thr[113]]$\beta$AR, [Phe[113]]$\beta$AR, [Ala[113]]$\beta$AR, nor [LYS[113]]$\beta$AR bound detectable levels of the antagonist.

The interactions of adrenergic ligands with mutant receptors which bind ligands with reduced affinity can be detected by measuring the ability of these compounds to act as agonists to mediate adenylyl cyclase stimulation by the mutant receptors. Thus, it has been found that [Glu[113]]$\beta$AR and [Asn[113]]$\beta$AR, which do not bind $^{125}$I-CYP with an affinity high enough to be detected by equilibrium binding measurements, are able to interact at low affinity with $\beta$-adrenergic agonists to stimulate adenylyl cyclase activity. The interactions of mutant receptors with Asn, Glu, His, or Lys at position 113 with several different compounds were examined by this method, with the results summarized in Tables 1 and 2. The agonist isoproterenol binds to and activates the wild-type receptor with a $K_{act}$ of $2 \times 10^{-8}$ M. The affinity of isoproterenol for the receptor is decreased 5000-fold by the substitution of an Asn residue for Asp[113] (Table 1) and 300-fold by the substitution of Glu for Asp[113] (Table 2). However, no stimulation of [Arg[113]]$\beta$AR, [Cys[113]]$\beta$AR, [His[113]]$\beta$AR, or [LYS[113]]$\beta$AR by isoproterenol was detected, even at ligand concentrations as high as 1 mM.

Substitution of the isopropyl group of isoproterenol with a guanidinium moiety to form 1-[2-(3,4-dihydroxyphenyl)-2-hydroxyethyl]guanidine (L-612,158) resulted in a 1000-fold decrease in affinity of the interaction with the wild type $\beta$AR. In addition, the efficacy of the interaction was reduced compared with isoproterenol, with the adenylyl cyclase stimulation obtained with the compound reaching a plateau at <20% the maximal level reached with NaF. This compound stimulated [Asn[113]]$\beta$AR with an affinity slightly higher than seen with the wild-type $\beta$AR (Table 1). Although the adenylyl cyclase activation mediated by L-612,158 activation of [Asn[113]]$\beta$AR did not reach a plateau at the experimentally accessible concentrations of the compound, the compound appeared to have nearly full agonist activity at this mutant receptor, with 50% maximal stimulation reached at the highest concentration of the ligand used.

TABLE 1

| Compound | [Asp113]$\beta$AR | [Asn113]$\beta$AR |
|---|---|---|
| | maximal adenylyl cyclase stimulation (% of NaF) ($K_{act}$) | |
| isoproterenol | 100% $2 \times 10^{-8}$ M | 100% $1 \times 10^{-4}$ M |
| L-612,158 | 11% $\leq 2 \times 10^{-5}$ M* | 50% $\leq 5 \times 10^{-5}$ M* |

LEGENDS

Table 1:

Adenylyl cyclase stimulation of mutant $\beta$ARs by compounds. Membranes from cell lines expressing the mutant receptors listed across the top of the table were incubated with the compounds listed and adenylyl cyclase stimulation measured, as described in Example 4. Data are presented as the % of NaF-mediated adenylyl cyclase stimulation achieved with a maximally effective concentration of the compound. In cases where a $K_{act}$ was determined, this value is listed below the % stimulation. N.d. means the value was not determined.

*In cases where the stimulation curve did not reach a plateau at experimentally assessible concentraions of a compound, Vmax was assumed to be equal to the maximal stimulation achieved with 10 mM NaF. The $K_{act}$ determined on the basis of this assumption represents the maximum value, and would be the true $K_{act}$ if the compound could, at high concentrations, act as a full agonist to fully activate the receptor. If the compound is in fact a partial agonist, then the actual $K_{act}$ would be lower than the calculated value.

EXAMPLE 7

## Stimulation or Inhibition of Adenylyl Cyclase Activity of Wild-type and Mutant AR by Adrenergic Ligands

Substitution of Asp[113] by Glu results in a mutant receptor which is partially activated by the compounds alprenolol and pindolol (Table 2). In contrast these compounds act as antagonists on the wild-type receptor. Cell lines expressing the wild-type $\beta$AR or [Glu[113]]$\beta$AR were developed and membranes prepared from these cell lines as previously described [Strader et al., P.N.A.S. (USA), 1987, supra; Dixon et al., EMBO 6, 3269 (1987); Strader et al., J. Bio. Chem. 262, 16439 (1987)]. Adenylyl cyclase activity was measured for 30 min at 30°C, using 20 mg of membrane protein in 50 ml of buffer containing $^{32}$P-ATP and an ATP regenerating system, as described previously [Strader et al., P.N.A.S. (USA), 1987, supra., Dixon et al., EMBO (1987) supra; Strader et al., J. Biol. Chem. (1987) supra; Saloman et al., supra. Activation of adenylyl cyclase was measured in the presence of the indicated concentrations of the activating ligands (Table 2), and data analyzed by computer-assisted nonlinear regressions according to the method of Example 5 which was described by Limbird, Cell Surface Receptors: A Short Course on Theory and Methods, Martinus Nijhoff, Boston, pp. 123-147 (1986). Ki values were determined from simultaneous analysis of 2-3 inhibition curves obtained at different concentrations of isoproterenol, according to the equation for competitive inhibition, $V = Vmax[A](Kact(1 + [B]/Ki) + [A])$, where B is the antagonist concentration and the other parameters are as described by Limbird, supra. The results are shown in Tables 2 & 3.

### TABLE 2

| | wild-type $\beta$AR | | [Glu[113]]$\beta$Ar | | [Asn[113]]$\beta$AR | |
|---|---|---|---|---|---|---|
| Compound | Kact | Ki(iso) | Kact | Ki(iso) | Kact | (Ki(iso)) |
| isoproterenol | $3.2\pm1.3\times10^{-8}$ | - | $8.0\pm.4\times10^{-6}$ | - | $2.3\pm1\times10^{-4}$ | - |
| alprenolol | - | $1.3\pm.3\times10^{-9}$ | $1.2\pm.4\times10^{-5}$ | $2.3\pm1.1\times10^{-4}$ | - | $2.8\pm.3\times10^{-5}$ |
| pindolol | - | $4.1\pm.5\times10^{-9}$ | $4.7\pm.5\times10^{-5}$ | $5.2\pm1.1\times10^{-5}$ | - | n.d. |
| propranolol | - | $2.8\pm.7\times10^{-9}$ | - | $2.4\pm1.7\times10^{-5}$ | - | $3.3\pm.6\times10^{-5}$ |

## TABLE 3

| COMPOUND | $(Asp^{113})\beta AR$ | | $(Glu^{113})\beta AR$ | |
|---|---|---|---|---|
| | %NaF | Kact(M) | %NaF | Kact(M) |
| (1) propanolol | 0 | n. d. | 7[*] | n. d. |
| (2) pindolol | 0 | n. d. | 35 | $4.7\pm5 \times 10^{-5}$ |
| (3) ICI-118 551 3-(7-methylindan-4-yloxy) -1-isopropylamino-2-propanol | 0 | n. d. | 6 | n. d. |
| (4) 3-phenoxy-1- isopropylamino-2-propanol | 0 | n. d. | 0 | n. d. |
| (5) 3-(4-(1-imidazol-2-yl)phenoxy)- 1-isopropylamino-2-propanol | 0 | n. d. | 0 | n. d. |

8

## TABLE 3 (cont'd)

| COMPOUND | (Asp[113]) βAR | | (Glu[113]) βAR | |
|---|---|---|---|---|
| | %NaF | Kact(M) | %NaF | Kact(M) |
| (6) atenolol | 0[+] | n. d. | 0[*] | n. d. |
| (7) metoprolol | 0[+] | n. d. | 2[*] | n. d. |
| (8) bisoprolol | 0[+] | n. d. | 0[*] | n. d. |
| (9) 3-o-morpholinophenoxy-1-isopropylamino-2-propanol | 0 | n. d. | 0 | n. d. |
| (10) oxyprenolol | 0 | n. d. | 31 | $\leq 5.3 \pm 6 \times 10^{-5}$ |

9

## TABLE 3 (cont'd)

| COMPOUND | $(Asp^{113})\beta AR$ | | $(Glu^{113})\beta AR$ | |
|---|---|---|---|---|
| | %NaF | Kact(M) | %NaF | Kact(M) |
| (11) alprenolol | 0 | n. d. | 30 | $1.2\pm4 \times 10^{-5}$ |
| (12) 3-(o-hydroxypehnoxy)-1-isopropylamino-2-propanol | 0 | n. d. | 0 | n. d. |
| (13) 3-(o-methoxyphenoxy)-1-isopropylamino-2-propanol | 0 | n. d. | 5* | n. d. |
| (14) 3-m-hydroxyphenoxy-1-isopropylamino-2-propanol | 8 | n. d. | 16 | $\leq 5.3\pm1.2 \times 10^{-4}$ |
| (15) 3-p-hydroxyphenoxy-1-isopropylamino-2-propanol | 9 | n. d. | 11 | n. d. |

## TABLE 3 (cont'd)

| COMPOUND | $(Asp^{113})\beta AR$ | | $(Glu^{113})\beta AR$ | |
|---|---|---|---|---|
| | %NaF | Kact(M) | %NaF | Kact(M) |
| (16) soterenol | 88 | $1.6\pm2 \times 10^{-7}$ | 76 | $6.0\pm4 \times 10^{-5}$ |
| (17) dichloroisoproterenol | 25 | $2.6\pm3 \times 10^{-7}$ | 16 | $2.1\pm4 \times 10^{-5}$ |
| (18) isoproterenol | 90 | $3.2\pm1.3 \times 10^{-8}$ | 95 | $8.0\pm4 \times 10^{-5}$ |
| (19) epinephrine | 95 | $8.0\pm.9 \times 10^{-8}$ | 85 | $1.2\pm.7 \times 10^{-5}$ |
| (20) norepinephrine | 95 | $1.1\pm.1 \times 10^{-6}$ | 50 | $\leq 1.3\pm2 \times 10^{-3}$ |

LEGEND

Table 3:

Stimulation of [Asp[113]]$\beta$AR (wild-type $\beta$AR) and [Glu[113]]$\beta$AR by adrenergic agonists and antagonists. The stimulation of adenylyl cyclase activity is presented as the percentage of the maximal stimulation by 10 mM NaF. Standard errors are <35% of mean values. The data shown were obtained from 10 separate experiments, during which the basal adenylyl cyclase activity of cells expressing [Glu[113]]$\beta$AR ranged from 4-7 pmol CAMP/mg/min and the NaF-stimulated activity ranged from 30-49 pmol/mg/min, corresponding to a stimulation by NaF of 6-9-fold above basal within any single experiment. For cells expressing the wild-type $\beta$AR, the ranges of basal and NaF-stimulated activity were 4-13 and 24-57 pmol cAMP/mg/min, respectively, corresponding to a stimulation of 4-12-fold within any single experiment. Compounds were tested at several concentrations, with a maximum concentration of $10^{-3}$ M, except as designated ($* = 10^{-4}$ M, $+ = 10^{-5}$ M). $K_{act}$ values were determined as described in Experimental Procedures for compounds showing stimulation of >11% of the value obtained with NaF. Stimulation lower than this was too low for reliable determination of a $K_{act}$ value. In cases where the activation curve did not reach a plateau at the

11

highest concentration of the compound tested, the actual efficacy of the ligand could not be determined from the data. In these cases, the $K_{act}$ values are presented as "<$K_{act}$" and were calculated on the assumption that the compound would be a full agonist at very high ligand concentrations. If the compound were only a partial agonist, the actual $K_{act}$ value would be lower than that stated. N.d. signifies that the value was not determined.

## EXAMPLE 8

The muscle-specific expression of luciferase in transgenic mice has been reported by DiLella et al., Nucleic Acids Research 16:4159 (1988). Using similar techniques an expression plasmid is prepared for the modified βAR receptor containing mutations at the codon for amino acid 113 as previously described. This plasmid contains a skeletal muscle specific promoter, muscle actin, so that, due to the tissue specific expression of the promoter, the βAR is expressed only in skeletal muscles. The plasmid is prepared by substituting the muscle actin promoter for the promoter in pSVL. The plasmid is injected into embryos of the selected avian or mammalian species, e.g., chickens, ducks and turkeys, or pigs, cattle and sheep, which are then transplanted into the mother or a surrogate. Progeny are screened for presence of the input plasmid DNA using oligonucleotide hybridization for the specific mutant βAR gene (Dixon et al., Nature, 1987, supra). DNA positive individuals are assayed by immunoblotting for expression of the BA, protein (Strader et al., Proc. Natl. Acad. Sci. USA, supra). Animals demonstrating expression of the βAR protein are cross-bred to produce stable populations of expressing animals. Animal lines expressing the mutated βAR are established and tissues from the animals are assayed for adenylyl cyclase responses to compounds specific for βAR and each of the mutants. Where positive responses are obtained, the animals are treated with ligands specified for that mutant βAR and in vivo responses measured.

Alternatively, a plasmid containing the RSV-LTR promoter, as reported by Overbeak et al., Science, 231: 1574 (1986) to direct muscle-specific expression of the βAR protein is used, with other procedures remaining as described above.

## Claims

1. A method of producing transgenic animals whose growth and metabolic characteristics can be favorably controlled by a method which comprises introducing into the genetic line of such animals a mutant β-adrenergic receptor whose activation requires the administration of a ligand specifically able to stimulate this receptor without affecting normal β-adrenergic receptors in the transgenic animal or other species.

2. A vector comprising a β-adrenergic receptor modified by replacement of the aspartic acid residue at position 113 by arginine, asparagine cysteine, glutamic acid, histidine, glutamine, serine, threonine, phenylalanine, alanine or lysine operatively linked to a muscle specific promoter.

3. A vector according to Claim 2 wherein the promoter is muscle actin.

4. A vector according to Claim 2 wherein the promoter is RSV-LTR.

5. An embryo of a member of an avian or mammalian human food species that has been transformed by incorporation of the vector of Claim 2.

6. An embryo of a member of an avian or mammalian human food species that has been transformed by incorporation of the vector of Claim 3.

7. An embryo according to Claim 6 wherein the species is chicken, duck or turkey.

8. An embryo according to Claim 5 wherein the species is cattle, pig or sheep.

9. The use of an agonist that does not react with a normal β-adrenergic receptor but which reacts with the modified receptor expressed by a transgenic member of an avian or a mammalian human food species that has been transformed with a vector of Claim 2, for the preparation of a composition useful for promoting growth in the transgenic member.

10. A member of an avian or a mammalian human food transgenic species having in its genome a β-adrenergic receptor modified by replacement of the aspartic acid residue at position 113 by arginine, asparagine, cysteine, glutamic acid, histidine, glutamine, serine, threonine, alanine, phenylalanine, or lysine.

11. A β-adrenergic receptor modified by replacement of the aspartic acid residue at position 113 by arginine, asparagine, cysteine, glutamic acid, histidine, glutamine, serine, threonine, phenylalanine, alaine or lysine.